# EUROPEAN PATENT SPECIFICATION

(11) **EP 3 507 299 B1**
(45) Date of publication and mention of the grant of the patent: **27.07.2022**
(21) Application number: 17847113.2
(22) Date of filing: 19.04.2017
(51) Int. Cl.: C07K 1/04

(54) **IN-SITU SOLVENT RECYCLING PROCESS FOR SOLID PHASE PEPTIDE SYNTHESIS AT ELEVATED TEMPERATURES**
IN-SITU-LÖSUNGSMITTELRECYCLINGVERFAHREN FÜR FESTPHASENPEPTIDSYNTHESE BEI ERHÖHTEN TEMPERATUREN
PROCÉDÉ DE RECYCLAGE DE SOLVANT IN SITU POUR LA SYNTHÈSE DE PEPTIDES EN PHASE SOLIDE À DES TEMPÉRATURES ÉLEVÉES

(30) Priority: 03.09.2016 US 201662383397 P; 21.10.2016 WO PCT/US2016/058181; 21.10.2016 US 201615299931; 18.04.2017 US 201715490090
(43) Date of publication of application: 10.07.2019
(73) Proprietor: CEM Corporation, Matthews, NC 28106 (US)
(72) Inventor: COLLINS, Jonathan, M., Charlotte, NC 28211 (US)
(74) Representative: Tomkins & Co
(86) International application number: PCT/US2017/028254
(87) International publication number: WO 2018/044356

(56) References cited:
- US-A1- 2014 275 481
- COLLINS, J. M. ET AL.: 'High-efficiency solid phase peptide synthesis (HE-SPPS' ORGANIC LETTERS, [Online] vol. 16, no. 3, 24 January 2014, pages 940 - 943, XP055274270 Retrieved from the Internet: <URL:http://pubs.acs.org/doi/abs/10.1021/0l 4036825>

### SEQUENCE LISTING

<110> CEM CORPORATION Collins, Jonathan
<120> IN-SITU SOLVENT RECYCLING PROCESS FOR SOLID PHASE PEPTIDE SYNTHESIS AT
   ELEVATED TEMPERATURES
<130> 1700.267
<140> 15/490,090 <141> 2017-04-18
<150> US 62/383,397
   <151> 2016-09-03
<160> 24
<170> PatentIn version 3.5
<210> 1
   <211> 27
   <212> PRT
   <213> Artificial Sequence
<220>
   <223> Synthesized
<400> 1
   Val Pro Leu Pro Ala Gly Gly Gly Thr Val Leu Thr Lys Met Tyr Pro
   1               5                   10                  15
   Arg Gly Asn His Trp Ala Val Gly His Leu Met
               20                  25
<210> 2
   <211> 29
   <212> PRT
   <213> Artificial Sequence
<220>
   <223> Synthesized
<400> 2
   His Ser Gln Gly Thr Phe Thr Ser Asp Tyr Ser Lys Tyr Leu Asp Ser
   1               5                   10                  15
   Arg Arg Ala Gln Asp Phe Val Gln Trp Leu Met Asn Thr
               20                  25
<210> 3
   <211> 20
   <212> PRT
   <213> Artificial Sequence
<220>
   <223> Synthesized
<400> 3
   Phe Pro Arg Pro Gly Gly Gly Gly Asn Gly Asp Phe Glu Glu Ile Pro
   1               5                   10                  15
   Glu Glu Tyr Leu
               20
<210> 4
   <211> 8
   <212> PRT
   <213> Artificial Sequence
<220>
   <223> Synthesized
<400> 4
   Asn Arg Val Tyr Val His Pro Phe
   1               5
<210> 5
   <211> 34
   <212> PRT
   <213> Artificial Sequence
<220>
   <223> Synthesized
<400> 5
   Ser Val Ser Glu Ile Gln Leu Met His Asn Leu Gly Lys His Leu Asn
   1               5                   10                  15
   Ser Met Glu Arg Val Glu Trp Leu Arg Lys Lys Leu Gln Asp Val His Asn Phe
               20                  25                  30
<210> 6
   <211> 10
   <212> PRT
   <213> Artificial Sequence
<220>
   <223> Synthesized
<400> 6
   Glu His Trp Ser Tyr Gly Leu Arg Pro Gly
   1               5                   10
<210> 7
   <211> 10
   <212> PRT
   <213> Artificial Sequence
<220>
   <223> Synthesized
<400> 7
   Glu His Trp Ser Tyr Trp Leu Arg Pro Gly
   1               5                   10
<210> 8
   <211> 31
   <212> PRT
   <213> Artificial Sequence
<220>
   <223> Synthesized
<400> 8
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Arg Gly Arg Gly
               20                  25                  30
<210> 9
   <211> 39
   <212> PRT
   <213> Artificial Sequence
<220>
   <223> Synthesized
<400> 9
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 10
   <211> 21
   <212> PRT
   <213> Artificial Sequence
<220>
   <223> Synthesized
<400> 10
   Met Glu Val Gly Trp Tyr Arg Ser Pro Phe Ser Arg Val Val His Leu
   1               5                   10                  15
   Tyr Arg Asn Gly Lys
               20
<210> 11
   <211> 26
   <212> PRT
   <213> Artificial Sequence
<220>
   <223> Synthesized
<400> 11
   His Asp Gly Thr Phe Thr Ser Glu Leu Ser Arg Leu Arg Asp Ser Ala
   1               5                   10                  15
   Arg Leu Gln Arg Leu Leu Gln Gly Leu Val
               20                  25
<210> 12
   <211> 34
   <212> PRT
   <213> Artificial Sequence
<220>
   <223> Synthesized
<400> 12
   Ser Val Ser Glu Ile Gln Leu Met His Asn Leu Gly Lys His Leu Asn
   1 5 10 15
   Ser Met Glu Arg Val Glu Trp Leu Arg Lys Lys Leu Gln Asp Val His
   20 25 30
   Asn Phe
<210> 13
   <211> 31
   <212> PRT
   <213> Artificial Sequence
<220>
   <223> Synthesized
<400> 13
   His Ala Glu Gly Thr Phe Thr Ser Asp Val Ser Ser Tyr Leu Glu Gly
   1 5 10 15
   Gln Ala Ala Lys Glu Phe Ile Ala Trp Leu Val Lys Gly Arg Gly
   20 25 30
<210> 14
   <211> 23
   <212> PRT
   <213> Artificial Sequence
<220>
   <223> Synthesized
<400> 14
   Gly Ile Gly Lys Phe Leu His Ser Ala Gly Lys Phe Gly Lys Ala Phe
   1 5 10 15
   Val Gly Glu Ile Met Lys Ser
   20
<210> 15
   <211> 24
   <212> PRT
   <213> Artificial Sequence
<220>
   <223> Synthesized
<400> 15
   Ser Tyr Ser Met Glu His Phe Arg Trp Gly Lys Pro Val Gly Lys Lys
   1 5 10 15
   Arg Arg Pro Val Lys Val Tyr Pro
   20
<210> 16
   <211> 9
   <212> PRT
   <213> Artificial Sequence
<220>
   <223> Synthesized
<400> 16
   Cys Tyr Phe Gln Asn Cys Pro Arg Gly
   1 5
<210> 17
   <211> 76
   <212> PRT
   <213> Artificial Sequence
<220>
   <223> Synthesized
<400> 17
<210> 18
   <211> 19
   <212> PRT
   <213> Artificial Sequence
<220>
   <223> Synthesized
<400> 18
   Lys Gly Arg Gly Lys Gln Gly Gly Lys Val Arg Ala Lys Ala Lys Thr
   1 5 10 15
   Arg Ser Ser
<210> 19
   <211> 17
   <212> PRT
   <213> Artificial Sequence
<220>
   <223> Synthesized
<400> 19
   Tyr Gly Gly Phe Leu Arg Arg Ile Arg Pro Lys Leu Lys Trp Asp Asn
   1 5 10 15
   Gln
<210> 20
   <211> 10
   <212> PRT
   <213> Artificial Sequence
<220>
   <223> Synthesized
<400> 20
   Val Gln Ala Ala Ile Asp Tyr Ile Asn Gly
   1 5 10
<210> 21
   <211> 25
   <212> PRT
   <213> Artificial Sequence
<220>
   <223> Synthesized
<400> 21
   Tyr Gly Gly Phe Met Arg Arg Val Gly Arg Pro Glu Trp Trp Met Asp
   1               5                   10                  15
   Tyr Gln Lys Arg Tyr Gly Gly Phe Leu
               20                  25
<210> 22
   <211> 11
   <212> PRT
   <213> Artificial Sequence
<220>
   <223> Synthesized
<400> 22
   Tyr Gly Arg Lys Lys Arg Arg Gln Arg Arg Arg
   1               5                   10
<210> 23
   <211> 13
   <212> PRT
   <213> Artificial Sequence
<220>
   <223> Synthesized
<400> 23
   Gly Arg Lys Lys Arg Arg Gln Arg Arg Arg Pro Pro Gln
   1               5                   10
<210> 24
   <211> 37
   <212> PRT
   <213> Artificial Sequence
<220>
   <223> Synthesized
<400> 24
   Lys Cys Asn Thr Ala Thr Cys Ala Thr Gln Arg Leu Ala Asn Phe Leu
   1               5                   10                  15
   Val His Ser Ser Asn Asn Phe Gly Pro Ile Leu Pro Pro Thr Asn Val
               20                  25                  30
   Gly Ser Asn Thr Tyr
           35

## Description

### Related Applications

This application is a continuation in part of Serial No. 15299931, filed October 21, 2016, for "Improvements in Solid Phase Peptide Synthesis."

### Background

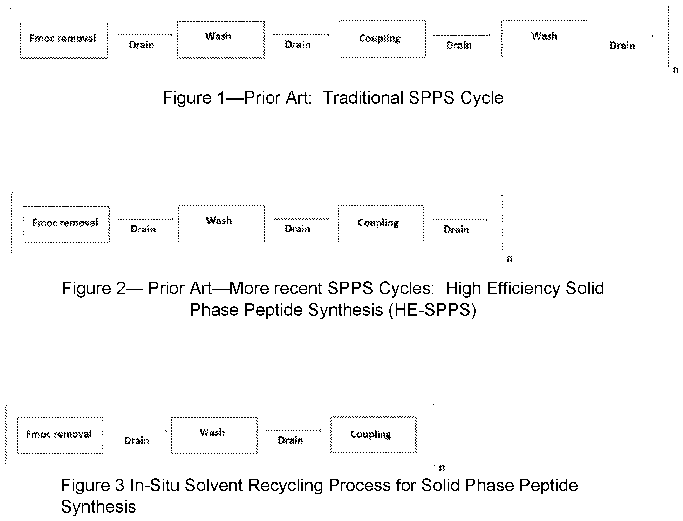

Bruce Merrifield's pioneering development of solid phase peptide synthesis created a useful process for synthesis peptide chains through its use of filtration to remove reagents between steps. The process has involved repetitive cycles which include coupling and deprotection with washing and filtration in-between each step (Figure 1).

It has commonly been assumed that washing is required between each step to completely remove the reagents previously used so that they don't undesirably participate in the next step. This typically involves "insertions" which refer to the incorporation of an extra amino acid. This is thought to occur through either residual base removing the protecting group (Fmoc) on an amino acid recently coupled thereby allowing a second amino acid to "insert"; or through residual activated amino acid left behind during the subsequent deprotection step which could couple to deblocked sites thereby "inserting" an extra amino acid from the previous step. It was recently shown, however, that washing after the coupling step was not required for the successful synthesis of peptides. In this work the coupling step was drained and the deprotection solution was subsequently added to the vessel (J. Collins, K. Porter, S. Singh and G. Vanier, "High-Efficiency Solid Phase Peptide Synthesis (HE-SPPS)," Org. Lett., vol. 16, pp. 940-943, 2014) (Figure 2).

### Summary

The invention is a method of deprotection in solid phase peptide synthesis in which the improvement comprises adding the deprotection composition in high concentration and small volume to the mixture of the coupling solution, the growing peptide chain, and any excess activated amino acid from the preceding coupling cycle; and without any draining step between the coupling step of the previous cycle and the addition of the deprotection composition for the successive cycle; and with the coupling solution at a temperature of at least 30°C.

In another aspect the invention is a method of deprotection in solid phase peptide synthesis in which the improvement comprises adding the deprotection composition in high concentration and small volume to the mixture of the coupling solution, the growing peptide chain, and any excess activated amino acid from the preceding coupling cycle; and without any draining step between the coupling step of the previous cycle and the addition of the deprotection composition for the successive cycle which removes at least 50% of the volume of the previous cycle coupling solution; and with the coupling solution at a temperature of at least 30°C.

The foregoing and other objects and advantages of the invention and the manner in which the same are accomplished will become clearer based on the followed detailed description taken in conjunction with the accompanying drawings.

### Brief Description of the Drawings

Figure 1 illustrates a traditional SPPS Cycle
Figure 2 illustrates more recent SPPS Cycles for High Efficiency Solid Phase Peptide Synthesis (HE-SPPS)
Figure 3 illustrates in-situ solvent recycling process for solid phase peptide synthesis.

### Detailed Description

This invention presents a novel process whereby the coupling and deprotection steps occur within the same solvent. In this process concentrated organic base is added directly to the resin coupling solution after a desired period of time for the coupling to occur. The deprotection step is then immediately started when the base is added. Therefore, the onset of the deprotection step is immediately after the coupling step without any time delay.

Additionally, only a small volume of base is required since it can use the solvent present from the coupling reaction. This requires a sophisticated reagent delivery system for the base that is accurate at very small volumes (0.5mL) with rapid delivery. Typically, a 20% solution of base (piperidine) in solvent is used for the deprotection step. Excess base concentration can increase base-catalyzed side reactions and therefore significant solvent is required. This means that significant solvent can be saved from this process by adding concentrated base to the coupling solvent.

To demonstrate the effectiveness of this new process a batch of 24 peptides were assembled using an automated peptide synthesizer modified to perform the in-situ solvent recycling step during each cycle.

### Materials and methods:

All peptides were synthesized using a LIBERTY BLUE^{™} PRIME^{™} system (CEM Corp., Matthews, NC, USA) allowing for automated in-situ solvent recycling and evaporation based washing. The peptides were synthesized at 0.05mmol scale with 10 equivalents of amino acid using CarboMAX^{™} coupling with amino acid/carbodiimide/ ethyl 2-cyano-2-(hydroxyimino)acetate (AA/DIC/Oxyma) (1:2:1) based activation for 100 sec at 90°C (E. Atherton, N. L. Benoiton, E. Brown, R. Sheppard and B. J. Williams, "Racemization of Activated, Urethane-protected Amino-acids by p-Dimethylaminopyridine. Significance in Solid Phase Peptide Synthesis," J.C.S. Chem. Comm., pp. 336-337, 1981). ProTide resins (CEM Corp.) based on TentaGel^{®} technology were used for synthesis with either a Rink Amide linker or a Cl-TCP(Cl) linker with unactivated loading of the first amino acid with DIEA at 90°C for 5 min. The deprotection step was performed for 50 sec at 95°C and initiated by adding 0.5mL of 50% pyrrolidine directly to the coupling solution. A single 1×4mL wash was used in between the deprotection and coupling steps. Peptides were cleaved with Trifluoroacetic acid (TFA)/triisopropylsilane/water/2,2'-(ethylenedioxy)diethanethiol (TFA/TIS/H₂O/DODt) (92.5:2.5:2.5:2.5) for 30 min at 38°C using a RAZOR^{™} cleavage system (CEM Corp.).

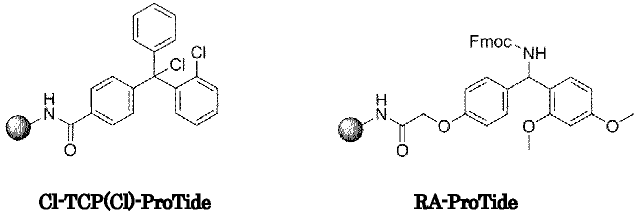

Results and discussion:

**Table 1 Automated Sequential Batch Synthesis of 24 Peptides**

| **#** | **Peptide** | **Disease Area** | **Resin Used** | **UPLC Purity (%)** | **Synthesis Time** |
|---|---|---|---|---|---|
| 1 | **GRP** (SEQ ID NO: 1) VPLPAGGGTVLTKMYPRGNHWAVGHLM-NH₂ | Regulates Gastrin Release | RA ProTide | 81 | 1:22 |
| 2 | **Glucagon** | Hypoglycemia | RA ProTide | 75 | 1:28 |
| 3 | **Bivalirudin** (SEQ ID NO: 3) H-**f**PRPGGGGNGDFEEIPEEYL-OH | Blood thinner | Cl-2-Cl-Trt | 71 | 1:05 |
| 4 | **Angiotensin** ( SEQ ID NO: 4) H-NRVYVHPF-OH | Vasoconstrictor | Cl-2-Cl-Trt | 82 | 0:30 |
| 5 | **PTH 1-84** | Osteoporosis | RA ProTide | 70 | 1:43 |
| 6 | **Gonadorelin** (SEQ ID NO: 6) pEHWSYGLRPG-NH₂ | Fertility | RA ProTide | 91 | 0:35 |
| 7 | **Triptorelin** (SEQ ID NO: 7) pEHWSYwLRPG-NH₂ | Breast Cancer, Prostrate Cancer, Fertility | RA ProTide | 73 | 0:35 |
| 8 | **Liraglutide** | Diabetes | RA ProTide | 80 | 1:31 |
| 9 | **Exenatide** | Diabetes | RA ProTide | 74 | 1:58 |
| 10 | **MOG (35-55)** | Multiple Sclerosis | RA ProTide | 71 | 1:05 |
| 11 | **Secretin** | Osmoregulatio n | RA ProTide | 70 | 1:19 |
| 12 | **Teriparatide** | Osteoporosis | RA ProTide | 60 | 1:43 |
| 13 | **GLP-1 (7-87)** | Diabetes | RA ProTide | 74 | 1:34 |
| 14 | **Magainin 1** | Antibiotic | RA ProTide | 79 | 1:11 |
| 15 | **Tetracosactide** | Adrenal Cortex stimulant | RA ProTide | 77 | 1:13 |
| 16 | **[Arg8]-Vasopressin** (SEQ ID NO: 16) H-CYFQNCPRG-NH₂ | Hormone (blood vessel contraction) | RA ProTide | 94 | 0:32 |
| 17 | **Ubiquitin** | Protein signaling agent | RA ProTide | ≥ 60 | 3:44 |
| 18 | **Parasin I** | Antibiotic | RA ProTide | 87 | 0:59 |
| 19 | **Dynorphin A** (SEQ ID NO: 19) H-YGGFLRRIRPKLKWDNQ-NH₂ | Opioid Research | RA ProTide | 71 | 0:53 |
| 20 | **ACP** (SEQ ID NO: 20) H-VQAAIDYING-NH₂ | Fatty Acid Synthesis | RA ProTide | 92 | 0:32 |
| 21 | **BAM 3200** | Opioid Research | RA ProTide | 70 | 1:16 |
| 22 | **HIV-TAT (47-57)** (SEQ ID NO: 22) Fmoc-YGRKKRRQRRR-NH₂ | HIV/AIDS Research | RA ProTide | 93 | 0:31 |
| 23 | **HIV-TAT (48-60)** (SEQ ID NO: 23) Fmoc-GRKKRRQRRRPPQ-NH₂ | HIV/AIDS Research | RA ProTide | 88 | 0:39 |
| 24 | **Pramlintide** | Diabetes | RA ProTide | 72 | 1:52 |

All peptides synthesized in Table 1 gave the desired target as the major peak with a standard cycle time of 2 minutes and 58 seconds. The in-situ solvent recycling process allowed for 0.5mL of a concentrated pyrrolidine (BP 87°C) solution to be added to the end of the coupling step (without draining). An advantage of this setup was that the deprotection immediately proceeded very close to the desired temperature (95°C) because the coupling solution was already at 90°C. During the deprotection process a vacuum was applied and the pyrrolidine was evaporated and subsequently condensed in the waste container. This allowed only a single wash step (1 x 4mL) to be required at the end of the deprotection step.

### Total synthesis time for entire batch: 32.6 hours

This new process provided a significant reduction in standard cycle time (2 minutes 57 seconds) from (a) - elimination of the coupling drain time, (b) - elimination of the deprotection delivery time between steps, and (c) - elimination of the temperature ramp time for the deprotection step thereby allowing a shorter deprotection time to be used. Additionally, significant solvent savings were possible with the complete elimination of the deprotection solvent during each cycle.

In the drawings and specification there has been set forth a preferred embodiment of the invention, and although specific terms have been employed, they are used in a generic and descriptive sense only and not for purposes of limitation, the scope of the invention being defined in the claims.

## Claims

1. A method of deprotection in solid phase peptide synthesis in which the improvement comprises:
adding an organic base deprotection composition to a mixture of a coupling solution, a growing peptide chain, and any excess activated amino acid from a preceding coupling step;
wherein the deprotection composition has a concentration of at least 50% base by volume of the deprotection composition and is added to the mixture of the coupling solution, the growing peptide chain, and any excess activated amino acid from the preceding coupling step in an amount that is less than 1/3 of the volume of the coupling solution;
without any draining step between the coupling step of the preceding coupling cycle and the addition of the deprotection composition for the successive cycle; and
with the coupling solution at a temperature of at least 30°C.

2. A method according to claim 1 wherein the deprotection composition is an organic base.

3. A method according to claim 1 using Fmoc solid phase peptide chemistry.

4. A method of deprotection in solid phase peptide synthesis in which the improvement comprises:
adding an organic base deprotection composition to a mixture of a coupling solution, a growing peptide chain, and any excess activated amino acid from the preceding coupling cycle step;
wherein the deprotection composition is added to the mixture of the coupling solution, the growing peptide chain, and any excess activated amino acid from the preceding coupling step in a concentration of at least 50% base by volume of the deprotection composition and an amount that is less than 1/3 of the volume of the coupling solution;
without any draining step between the coupling step of the preceding coupling cycle and the addition of the deprotection composition for the successive cycle which removes at least 50% of the volume of the preceding coupling cycle coupling solution; and
with the coupling solution at a temperature of at least 30°C.

5. A method according to claim 4 wherein the deprotection composition is an organic base.

6. A method according to claim 4 using Fmoc solid phase peptide chemistry.

## Patentansprüche

1. Verfahren zum Entschützen bzw. Entfernen einer Schutzgruppe bei einer Festphasensynthese von Peptiden, wobei die Verbesserung Folgendes aufweist:
Hinzufügen einer Entschützungsverbindung mit einer organischen Lauge zu einer Mischung einer Koppelungslösung, einer wachsenden Peptidkette und jeglichen Überschuss aktivierter Aminosäure aus einem vorhergehenden Koppelungsschritt;
wobei die Entschützungsverbindung eine Konzentration von mindestens 50 Volumen-% Lauge der Entschützungsverbindung hat und zu der Mischung der Koppelungslösung, der wachsenden Peptidkette und
jeglichem Überschuss aktivierter Aminosäure von dem vorhergehenden Koppelungsschritt in einer Menge zugegeben wird, die weniger als 1/3 des Volumens der Koppelungslösung ist;
und zwar ohne einen Ablassschritt zwischen dem Koppelungsschritt des vorhergehenden Koppelungsschrittes und der Zugabe der Entschützungsverbindung für den nachfolgenden Zyklus; und
wobei die Koppelungslösung auf einer Temperatur von mindestens 30 °C ist.

2. Verfahren nach Anspruch 1, wobei die Entschützungsverbindung eine organische Lauge ist.

3. Verfahren nach Anspruch 1, welches Fmoc-Festphasenpeptidchemie verwendet.

4. Verfahren zum Entschützen bzw. Entfernen einer Schutzgruppe bei einer Festphasensynthese von Peptiden, wobei die Verbesserung Folgendes aufweist:
Hinzufügen einer Entschützungsverbindung mit einer organischen Lauge zu einer Mischung einer Koppelungslösung, einer wachsenden Peptidkette und jeglichem Überschuss aktivierter Aminosäure aus einem vorhergehenden Koppelungszyklusschritt;
wobei die Entschützungsverbindung zu der Mischung der Koppelungslösung, der wachsenden Peptidkette und jeglichem Überschuss aktivierter Aminosäure von dem vorhergehenden Koppelungsschritt in einer Konzentration von mindestens 50 Volumen-% Lauge der Entschützungsverbindung und einer Menge hinzugegeben wird,
die geringer ist als 1/3 des Volumens der Koppelungslösung;
und zwar ohne einen Ablassschritt zwischen dem Koppelungsschritt des vorhergehenden Koppelungszyklus und der Zugabe der Entschützungsverbindung für den nachfolgenden Zyklus, wobei mindestens 50 Volumen-% der Koppelungslösung des vorhergehenden Koppelungszyklus entfernt wird; und
wobei die Koppelungslösung auf einer Temperatur von mindestens 30 °C ist.

5. Verfahren nach Anspruch 4, wobei die Entschützungsverbindung eine organische Lauge ist.

6. Verfahren nach Anspruch 4, welches Fmoc-Festphasenpeptidchemie verwendet.

## Revendications

1. Un procédé de déprotection dans la synthèse peptidique en phase solide dans lequel l'amélioration comprend :
l'ajout d'une composition de déprotection à base organique à un mélange d'une solution de couplage, d'une chaîne peptidique en croissance et de tout excès d'acide aminé activé provenant d'une étape de couplage précédente;
dans lequel la composition de déprotection présente une concentration d'au moins 50 % de la base par volume de la composition de déprotection et est ajoutée au mélange de la solution de couplage, de la chaîne peptidique en croissance et de tout excès d'acide aminé activé de l'étape de couplage précédente en une quantité qui est inférieure à 1/3 du volume de la solution de couplage;
sans étape de vidange entre l'étape de couplage du cycle de couplage précédent et l'addition de la composition de déprotection pour le cycle suivant; et
avec la solution de couplage à une température d'au moins 30°C.

2. Un procédé selon la revendication 1, dans lequel la composition de déprotection est une base organique.

3. Un procédé selon la revendication 1, utilisant la chimie des peptides en phase solide Fmoc.

4. Un procédé de déprotection dans la synthèse peptidique en phase solide dans lequel l'amélioration comprend :
l'ajout d'une composition de déprotection de base organique à un mélange d'une solution de couplage, d'une chaîne peptidique en croissance et de tout excès d'acide aminé activé provenant de l'étape de cycle de couplage précédente;
dans lequel la composition de déprotection est ajoutée au mélange de la solution de couplage, de la chaîne peptidique en croissance et de tout excès d'acide aminé activé de l'étape de couplage précédente à une concentration d'au moins 50 % de base par volume de la composition de déprotection et une quantité qui est inférieure à 1/3 du volume de la solution de couplage;
sans étape de vidange entre l'étape de couplage du cycle de couplage précédent et l'addition de la composition de déprotection pour le cycle successif qui élimine au moins 50% du volume de la solution de couplage du cycle de couplage précédent; et
avec la solution de couplage à une température d'au moins 30°C

5. Un procédé selon la revendication 4, dans lequel la composition de déprotection est une base organique.

6. Un procédé selon la revendication 4, utilisant la chimie des peptides en phase solide Fmoc.
